(19) Europäisches Patentamt
European Patent Office
Office européen des brevets

(11) Veröffentlichungsnummer: **0 144 798**
**B1**

# (12) EUROPÄISCHE PATENTSCHRIFT

(45) Veröffentlichungstag der Patentschrift: **27.06.90**

(21) Anmeldenummer: **84113533.8**

(22) Anmeldetag: **09.11.84**

(51) Int. Cl.[5]: **C 12 Q 1/48,** C 12 Q 1/34, C 12 Q 1/26, C 12 Q 1/28, C 12 Q 1/32

## (54) Verfahren und Reagenz zur Bestimmung von Glycerin mittels eines kompetitiven Inhibitors für Glycerinkinase.

(30) Priorität: **10.11.83 DE 3340709**

(43) Veröffentlichungstag der Anmeldung: **19.06.85 Patentblatt 85/25**

(45) Bekanntmachung des Hinweises auf die Patenterteilung: **27.06.90 Patentblatt 90/26**

(84) Benannte Vertragsstaaten: **AT BE CH DE FR GB IT LI LU NL SE**

(56) Entgegenhaltungen:
**EP-A-0 030 718**
**EP-A-0 044 615**
**EP-A-0 045 031**
**FR-A-2 336 682**
**US-A-4 309 502**

(73) Patentinhaber: **BOEHRINGER MANNHEIM GMBH**
**Patentabteilung, Abt. E Sandhofer Strasse 112-132 Postfach 31 01 20**
**D-6800 Mannheim 31 Waldhof (DE)**

(72) Erfinder: **Willnow, Peter, Dr. rer. nat.**
**Eichenstrasse 29**
**D-8139 Bernried (DE)**
Erfinder: **Lehmann, Paul, Dr. rer. nat.**
**Lange Strasse 28**
**D-8132 Tutzing (DE)**
Erfinder: **Ziegenhorn, Joachim, Dr. rer. nat.**
**Ina-Seidel-Weg 1**
**D-8130 Starnberg (DE)**
Erfinder: **Wahlefeld, August Wilheim, Dr. rer. nat.**
**Alpenblickstrasse 25**
**D-8126 Hohenpeissenberg (DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 144 798 B1

Courier Press, Leamington Spa, England.

## Beschreibung

Die Erfindung betrifft ein Verfahren und ein Reagenz zur Bestimmung von Glycerin unter Verwendung von Glycerinkinase.

Es ist bekannt, Glycerin, gegebenenfalls nach vorheriger Freisetzung aus seinen Estern durch chemische oder enzymatische Verseifung, durch Umsetzung mit ATP in Gegenwart von Glycerinkinase (GK) Bildung von Glycerin-3-phosphat und ADP und Messung eines dieser Reaktionsprodukte mit Hilfe mindestens einer nachgeschalteten enzymatischen Reaktion zu bestimmen (H. U. Bergmeyer in "Methoden der enzymatischen Analyse", 3. Auflage, S. 1448 bzw. 1872). Diese enzymatische Glycerin-Bestimmungsverfahren hat zwar aufgrund seiner Spezifität gegenüber den früher gebräuchlichen chemischen Bestimmungsmethoden einen erheblichen Fortschritt gebracht, jedoch eignet sich das Verfahren bisher nicht zur raschen und praktikablen kinetischen Durchführung. Aufgrund des niedrigen $K_M$-Werts der Glycerinkinase, die den spezifischsten Teilschritt der Reaktionsfolge katalysiert, verläuft die Reaktion in dem für die Glycerinbestimmung interessanten Konzentrationsbereich nicht nach erster bzw. pseudoerster Ordnung. Ein derartiger Reaktionsverlauf ist jedoch Voraussetzung für eine rasche, praktikable, keinen Probenleerwert erfordernde kinetische Bestimmungsmethode, die eine wesentliche Verkürzung des Zeitbedarfs pro Einzelanalyse gegenüber den bisher erforderlichen Endpunkt- bzw. kinetischen Methoden ermöglichen würde. Dabei lag der Zeitbedarf für die bisherigen photometrischen Verfahren zwischen 10 und 6 Minuten; angestrebt werden bei kinetischen Methoden Analysenzeiten von 1 bis 3 Minuten. Die Analysenautomaten der neuen Generation sind für einen hohen Probendurchsatz konzipiert und gestatten nur kurze Inkubationszeiten, die mit den bisherigen Verfahren, im Rahmen des Glycerinnachweises, nicht erzielt werden können. Infolgedessen können die heute üblichen Analysenautomaten mit hoher Analysenfrequenz nicht voll ausgelastet werden.

Der Erfindung liegt daher die Aufgabe zugrunde, ein kinetisches Bestimmungsverfahren für die Glycerinkinase-Reaktion zu entwickeln, bei dem diese nach pseudoerster Ordnung abläuft.

Es ist bekannt, daß es in vielen Fällen möglich ist, einen derartigen Reaktionsverlauf bei zu niedrigen $K_M$-Werten der beteiligten Enzyme durch künstliche $K_M$-Werterhöhung zu erzielen. Aus der Theorie von Michaelis und Menten folgt, daß enzymkatalysierte Einsubstrat-Reaktionen dann über einen weiten Konzentrationsbereich nach erster Ordnung ablaufen, wenn die Michaelis-Konstante des Enzyms sehr viel größer ist als die maximale Substratkonzentration. Da die bisher bekannten Glycerinkinasen aus verschiedenen Mikroorganismengattungen, wie Bacillus, Escherichia coli, Candida, Cellulomonas, Streptomyces oder Hefe $K_M$-Werte (Glycerin) in der Größenordnung von $10^{-4}$ bis $10^{-5}$ mol/l aufweisen, lassen sich hier nur geringe Glycerinkonzentrationen kinetisch messen. Es ist zwar bekannt, daß man durch Zusatz eines kompetitiven Inhibitors den $K_M$-Wert eines Enzyms künstlich erhöhen kann, ein geeigneter kompetitiver Inhibitor für die GK ist jedoch nicht bekannt.

Der Erfindung liegt daher die Aufgabe zugrunde, ein Verfahren und ein Reagenz zu schaffen, welches eine kinetische Glycerinbestimmung im Rahmen eines mehrstufigen enzymatischen Verfahrens ermöglicht und sich auch für Analysenautomaten hoher Frequenz eignet. Das Verfahren sollte weiter als Farbtest durchführbar sein und auch auf Teststreifen anwendbar sein.

Gelöst wird diese Aufgabe erfindungsgemäß durch ein Verfahren zur Bestimmung von Glycerin in freier oder gebundener Form durch Umsetzung mit ATP in Gegenwart von Glycerinkinase (GK) und gegebenenfalls einer Hydrolase unter Bildung von Glycerin-3-Phosphat und ADP und Bestimmung eines dieser Reaktionsprodukte mit Hilfe mindestens einer nachgeschalteten enzymatischen Reaktion, welches dadurch gekennzeichnet ist, daß die Bestimmung kinetisch durchgeführt und hierzu die Umsetzung mit ATP für die Gesamtreaktion geschwindigkeitsbestimmend gemacht und nach pseudoerster Ordnung ablaufen gelassen wird, indem man einen Zucker der allgemeinen Formel I zusetzt

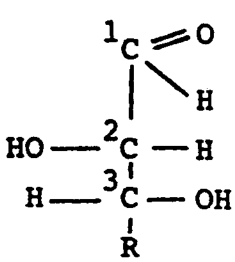

in der die C-Atome 2 und 3 D-threo-Konfiguration aufweisen und R einen Kohlenhydratrest mit 1 bis 3 Kohlenstoffatomen darstellt, bei dem eine OH-Gruppe auch durch ein Wasserstoffatom ersetzt sein kann.

Die Erfindung beruht auf der überraschenden Feststellung, daß Zucker der angegebenen allgemeinen Formel kompetitive Inhibitoren der GK sind und sich daher ausgezeichnet zur Veränderung der $K_M$ eignen.

Typische Beispiele für erfindungsgemäß verwendbare Inhibitoren sind D-Threose ($R=CH_2OH$), L-Xylose, D-Arabinose ($R=CHOH—CH_2OH$), L-Fucose ($R=CHOH—CHOH—CH_3$), L-Galactose, L-Glucose ($R=CHOH—CHOH—CH_2OH$). Bevorzugt wird L-Xylose verwendet.

Im Rahmen des erfindungsgemäßen Verfahrens setzt man einen Zucker der allgemeinen Formel I

zweckmäßig in Mengen zwischen 1 und 200 mMol/l ein. Die jeweils erforderliche Menge hängt einerseits von der angestrebten $K_M$, andererseits von der Menge an GK ab. Die GK selbst wird im allgemeinen in Mengen von $10^2$ bis $10^4$ U/l eingesetzt werden, obwohl auch geringere oder größere Mengen in Betracht kommen.

Die kinetische Bestimmung erfolgt in der dem Fachmann bekannten Weise, zweckmäßig indem man mindestens zwei Messungen in einem vorher festgelegten Zeitintervall durchführt. Die besten Ergebnisse werden dabei im pH-Wert-Bereich von etwa 7,5 bis etwa 8,5 erzielt, jedoch kann aus diesem Bereich je nach den durch die Hilfsenzyme sich ergebenden Bedingungen abgewichen werden.

Welche Hilfsenzyme weiter benötigt werden, hängt davon ab, welches Reaktionsprodukt der geschwindigkeitsbestimmenden Reaktion, Glycerin-3-Phosphat oder ADP, bestimmt werden soll. Gemäß einer bevorzugten Ausführung der Erfindung wird gebildetes Glycerin-3-Phosphat mit Glycerin-3-Phosphatoxidase in Dihydroxyacetonphosphat und $H_2O_2$ überführt und letzteres in üblicher Weise bestimmt. Diese Ausführungsform der Erfindung läßt sich wie folgt durch Reaktionsgleichungen veranschaulichen:

$$1)\ \text{Triglycerid} + 3H_2O \xrightarrow{\text{Esterase EC 3.1.1}} \text{Glycerin} + \text{Fettsäuren}$$

$$2)\ \text{Glycerin} + \text{ATP} \xrightarrow[\text{Verbindung v. Formel I}]{\text{GK EC 2.7.1.30}} \text{Glycerin-3-Phosphat} + \text{ADP}$$

$$3)\ \text{Glycerin-3-Phosphat} + O_2 \xrightarrow{\text{GPO}} \text{Dihydroxyaceton-Phosphat} + H_2O_2$$

Wie die obigen Formeln zeigen, bildet sich hierbei $H_2O_2$, welches sich beispielsweise mit Peroxidase in Gegenwart von Phenol und 4-Aminoantipyrin in GOOD-Puffer in einen direkt meßbaren Farbstoff überführen läßt.

Da die obige Umsetzung 3) unter Sauerstoffverbrauch verläuft, kann anstelle von $H_2O_2$ auch Dihydroxyacetonphosphat oder der Sauerstoffverbrauch gemessen werden.

Alternativ kann man gebildetes ADP messen. Dies kann beispielsweise erfolgen, indem man im obigen Reaktionsschema Gleichung 3) ersetzt durch die nachstehenden Gleichungen 4) und 5).

$$4)\ \text{ADP} + \text{PEP} \xrightarrow{\text{PK}} \text{Pyruvat} + \text{ATP}$$

$$5)\ \text{Pyruvat} + \text{NADH} \xrightarrow{\text{LDH}} \text{Lactat} + \text{NAD}^+$$

Hierbei kann im UV direkt die NADH-Abnahme verfolgt werden.

Bei dieser Bestimmung wird ADP mit Phosphoenolpyruvat in Gegenwart von Pyruvatkinase zu Pyruvat umgesetzt, welches wiederum mit NADH in Gegenwart von Lactatdehydrogenase zu Lactat und $NAD^+$ reagiert.

Gemäß einer weiteren Ausführungsform der Erfindung wird gebildetes Glycerin-3-Phosphat bestimmt durch Umsetzung mit $NAD^+$ in Gegenwart von Glycerinphosphatdehydrogenase unter Bildung von Dihydroxyacetonphosphat und NADH. Letzteres wird entweder direkt im UV oder durch Umsetzung mit einem Tetrazoliumsalz in Gegenwart von Diaphorase bestimmt, wobei in letzterem Fall der gebildete Formazan-Farbstoff gemessen wird.

Diese Umsetzung läßt sich durch die Reaktionsgleichungen 6) und 7) darstellen, die an die Stelle der Reaktionsgleichung 3) treten.

$$6)\ \text{NAD} + \text{Glycerin-3-Phosphat} \xrightarrow{\text{Glycerinphosphat-Dehydrogenase}} \text{NADH} + \text{Dihydroxyacetonphosphat}$$

$$7)\ \text{NADH} + \text{Tetrazoliumsalz} \xrightarrow{\text{Diaphorase}} \text{NAD}^+ + \text{Formazan}$$

Das erfindungsgemäße Verfahren kann vorteilhaft in Gegenwart eines nichtionischen Detergenz und gegebenenfalls zusätzlich eines Detergenz der Cholsäuregruppe durchgeführt werden.

Hinsichtlich der Quelle der eingesetzten Glycerinkinase bestehen keine Beschränkungen. Typische Beispiele für im Rahmen der Erfindung verwendbare Glycerinkinasen sind die Enzyme aus Bacillus stearothermophilus, E. coli, Candida mycoderma, Streptomyces canus, Cellulomonas species und Hefe. Aus anderen Quellen ist das Enzym derzeit nicht handelsüblich. Bevorzugt wird das Enzym aus Bacillus stearothermophilus verwendet.

Ein weiterer Gegenstand der Erfindung ist ein Reagenz zur kinetischen Bestimmung von Glycerin, enthaltend Glycerinkinase, und ein System zur Bestimmung von Glycerin-3-Phosphat oder ADP und gegebenenfalls eine Hydrolase, welches dadurch gekennzeichnet ist, daß es zusätzlich einen Zucker der allgemeinen Formel

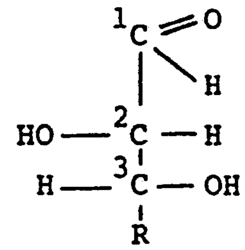

in der die C-Atome 2 und 3 D-threo-Konfiguration aufweisen und R einen Kohlenhydratrest mit 1 bis 3 Kohlenstoffatomen darstellt, bei dem eine OH-Gruppe auch durch ein Wasserstoffatom ersetzt sein kann, enthält.

Die Zusammensetzung des erfindungsgemäßen Reagenz wird qualitativ durch das jeweils eingesetzte System zur Bestimmung von Glycerin-3-phosphat oder ADP bestimmt. Für die quantitative Zusammensetzung gelten die oben im Zusammenhang mit dem Verfahren gemachten Ausführungen in gleicher Weise. So enthält das Reagenz vorzugsweise 1 bis 200 mMol/l Verbindung der allgemeinen Formel I. Die Bevorzugte Verbindung der allgemeinen Formel I ist L-Xylose.

Das System zur Bestimmung von Glycerin-3-phosphat besteht vorzugsweise entweder aus Glycerinphosphat-oxidase und einem System zur Bestimmung von $H_2O_2$ oder Dihydroxyacetonphosphat oder aus Glycerinphosphat-dehydrogenase, $NAD^+$, Tetrazoliumsalz und Diaphorase.

Das System zur Bestimmung von $H_2O_2$ besteht vorzugsweise aus 4-Aminoantipyrin, Phenol oder Phenolderivat, Puffer und Detergenz.

Enthält das erfindungsgemäße Reagenz ein System zur Bestimmung von ADP, so besteht letzteres wieder vorzugsweise aus Phosphoenolpyruvat, Pyruvatkinase, NADH und Lactatdehydrogenase.

In einer besonders bevorzugten Zussammensetzung besteht das erfindungsgemäße Reagenz entsprechend den Reaktionsgleichungen 1 bis 3 aus

$10^2$ bis $10^4$ U/l Glycerinkinase,
$10^2$ bis $10^4$ U/l Glycerinphosphatoxidase,
$10^3$ bis $2\times10^4$ U/l Cholesterin-Esterase,
$10^2$ bis $10^4$ U/l Peroxidase,
1 bis 20 mMol/l L-Xylose,
0,1 bis 1 mMol/l 4-Aminoantipyrin,
1 bis 10 mMol/l Phenol oder Phenol-Derivat,
1 bis 20 g/l nichtionisches Detergenz,
0 bis 15 mMol/l Detergenz der Cholsäuregruppe,
50 bis 200 mMol/l Puffer, pH 7,5 bis 8,5.

Als Puffersubstanzen lassen sich alle im angegebenen pH-Bereich wirksamen Puffer verwenden. Bevorzugt wird GOOD-Puffer.

Das erfindungsgemäße verfahren läßt sich auf den üblichen Analysengeräten und Aanlysenautomaten durchführen, wie in den Beispielen noch näher gezeigt wird. Das Reagenz kann auch auf einem festen Träger imprägniert vorliegen, beispielsweise auf einem Papierstreifen. Im letzteren Fall läßt sich die Farbbildung ebenfalls quantitativ kinetisch bestimmen.

Die Wirksamkeit des erfindungsgemäß verwendeten Inhibitors ergibt sich aus der beigefügten Zeichnung. In dieser stellen dar:

Fig. 1 eine graphische Darstellung der gemessenen Reaktionsgeschwindigkeiten ohne kompetitiven Hemmstoff, und mit verschiedenen Konzentrationen an Verbindung der Formel I (L-Xylose) bei dem Enzym aus Bac. stearothermophilus. Die Reagenzzusammensetzung war wie folgt:

2,0 U/ml GK,
1,5 U/ml POD,
10 U/ml GPO,
1 mMol/l ATP,
3,5 mMol/l 4-Chlorphenol,
0,35 mMol/l 4-Aminoantipyrin,
0,1 mol/l Tris-/HCl-Puffer, pH 7,6,
Temperatur 25°C; Messwellenlänge 546 nm.

Aus den Kurven ergibt sich eine $K_M$ ohne Hemmstoff≈0,052 mMol/l. Mit 5 mMol/l betrug die $K_M$ 0,24 mMol/l, mit 10 mMol/l Hemmstoff≈0,43 mMol/l.

Fig. 2 entspricht Fig. 1, jedoch wurde das Enzym aus E. coli verwendet. Die $K_M$ ohne Hemmstoff ergab

sich zu etwa 0,042 mMol/l, mit 10 mMol/l Hemmstoff zu 0,16 mMol/l und mit 30 mMol/l Hemmstoff zu 0,28 mMol/l.

Die Erfindung kann zur Messung von Glycerin bzw. der Triglyceride im Serum oder Plasma, beispielsweise Heparin- oder EDTA-Plasma verwendet werden.

Die folgenden Beispiele erläutern die Erfindung weiter:

Beispiel 1

Verwendetes Reagenz:

| | |
|---|---|
| Tris/HCl-Puffer | 0,15 Mol/l, pH 7,6 |
| $MgSO_4 \times 7H_2O$ | 17,5 mMol/l |
| EDTA, Dinatriumsalz | 10 mMol/l |
| 4-Chlorphenol | 3,5 mMol/l |
| Natriumcholat | 0,15% |
| Detergens | 0,12% |
| ATP | ≥0,5 mMol/l |
| 4-Aminoantipyrin | =0,35 mMol/l |
| Esterase | ≥3 U/ml |
| Glycerinphosphatoxidase | ≥2,5 U/ml |
| Glycerinkinase | ≥0,2 U/ml |
| Peroxidase | ≥0,15 U/ml |

Bestimmungsansatz:

| | |
|---|---|
| Wellenlänge: | Hg 546 Spektralphotometer: 500 nm |
| Küvette: | 1 cm Schichtdicke |
| Inkubationstemperatur: | 20 bis 25°C oder 37°C |
| Messung gegen RL: | (Extinktionszunahme) |

Für jede Meßreihe genügt ein Reagenzienleerwert (RL) und ein Standard.

| In Reagenzgläser pipettieren | | | |
|---|---|---|---|
| | RL | Probe | Standard |
| Probenmaterial | -- | 0,02 ml | - |
| Standard | -- | -- | 0,02 ml |
| Reagenzlösung | 2,00 ml | 2,00 ml | 2,00 ml |
| mischen, RL, Probe und Standard 10 Minuten bei 20 bis 25°C oder 37°C inkubieren. Innerhalb von 30 Minuten Extinktion der Probe gegen RL messen. | | | |

Die Verdünnungsgrenze liegt bei 1000 mg/dl bzw. 11,4 mMol/l. Bei höheren Konzentrationen Probe 1+5 mit Natriumchlorid-Lösung (0,9%) verdünnen und Bestimmung wiederholen: Ergebnis × 6.
Als Standard wird eine wässrige Glycerinlösung, entsprechend 200 mg/dl Triglyceride, eingesetzt.

Berechnung:

Auswertung über Standard:

$$c[mg/dl] = \frac{E\ Probe}{E\ Standard} \times 200$$

Auswertung über Faktor:

Die Konzentration der Triglyceride in der Probe berechnen nach:

| Meßwellenlänge | c [mg/dl] | c [mMol/l] |
|---|---|---|
| Hg 546 nm | 1040 x $E_{Probe}$ | 11,9 x $E_{Probe}$ |
| 500 nm | 760 x $E_{Probe}$ | 8,66 x $E_{Probe}$ |

Beispiele 2 bis 7

Vergleichende Untersuchung verschiedener erfindungsgemäßer Inhibitoren mit GK unterschiedlicher Herkunft:

Reagenzzusammensetzung:

| | |
|---|---|
| Tris/HCl | 0,1 mol/l, pH 7,6 |
| Glycerin-phosphat-oxidase | 10 U/ml |
| Peroxidase | 1,5 U/ml |
| Glycerinkinase | 2,0 U/ml |
| $MgSO_4 \times 7H_2O$ | 17,5 mMol/l |
| Inhibitor | siehe Tabellen |
| 4-Chlorphenol | 3,5 mMol/l |
| 4-Aminoantipyrin | 0,35 mMol/l |
| ATP | 1 mMol/l |

Probe: wässrige Glycerin-Lsg. 6 mMol/l

| | |
|---|---|
| Probe/Reagenz-Verhältnis: | 1:100 (20 µl/2 ml) |
| Temperatur: | 25°C |
| Meßwellenlänge: | 546 nm |

(ΔE/min jeweils aus dem linearen Bereich abgelesen)

Die Ergebnisse mit L-Xylose, L-Fucose und D-Arabinose bei dem Enzym aus Bac. stearothermophilus bzw. E. coli zeigen die nachstehenden Tabellen 1 und 2.

Tabelle 1

GK aus Bac. stearothermophilus

| L-Xylose [mMol/l] | ΔE/min | L-Fucose [mMol/l] | ΔE/min | D-Arabinose [mMol/l] | ΔE/min |
|---|---|---|---|---|---|
| -- | 0,515 | -- | 0,522 | -- | 0,509 |
| 2 | 0,320 | 10 | 0,230 | 10 | 0,426 |
| 5 | 0,188 | 20 | 0,153 | 20 | 0,349 |
| 10 | 0,105 | 50 | 0,077 | 50 | 0,249 |
| 20 | 0,062 | 100 | 0,039 | 100 | 0,121 |

Tabelle 2

GK aus E. coli

| L-Xylose [mMol/l] | ΔE/min | L-Fucose [mMol/l] | ΔE/min | D-Arabinose [mMol/l] | ΔE/min |
|---|---|---|---|---|---|
| -- | 0,420 | -- | 0,441 | -- | 0,435 |
| 5 | 0,228 | 20 | 0,352 | 20 | 0,363 |
| 10 | 0,169 | 50 | 0,286 | 50 | 0,302 |
| 20 | 0,118 | 100 | 0,209 | 100 | 0,231 |
| 50 | 0,072 | 150 | 0,142 | 150 | 0,167 |

**Patentansprüche**

1. Verfahren zur Bestimmung von Glycerin in freier oder gebundener Form durch Umsetzung mit ATP in Gegenwart von Glycerinkinase (GK) und gegebenenfalls einer Hydrolase unter Bildung von Glycerin-3-Phosphat und ADP und Bestimmung eines dieser Reaktionsprodukte mit Hilfe mindestens einer nachgeschalteten enzymatischen Reaktion, dadurch gekennzeichnet, daß die Bestimmung kinetisch durchgeführt und hierzu die Umsetzung mit ATP für die Gesamtreaktion geschwindigkeitsbestimmend gemacht und nach pseudo-erster Ordnung ablaufen gelassen wird, indem man einen Zucker der allgemeinen Formel I zusetzt

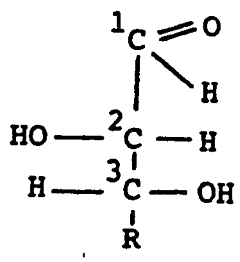

in der die C-Atome 2 und 3 D-threo-Konfiguration aufweisen und R einen Kohlenhydratrest mit 1 bis 3 Kohlenstoffatomen darstellt, bei dem eine OH-Gruppe auch durch ein Wasserstoffatom ersetzt sein kann.

2. Verfahren nach Anspruch 1, dadurch gekennzeichnet, daß man 1 bis 200 mMol/l der Verbindung von Formel 1 zusetzt.

3. Verfahren nach Anspruch 1 oder 2, dadurch gekennzeichnet, daß man als Verbindung der allgemeinen Formel I L-Xylose verwendet.

4. Verfahren nach einem der vorhergehenden Ansprüche, dadurch gekennzeichnet, daß man eine Glycerinkinase aus einem Mikroorganismus der Gattung Bacillus stearothermophilus verwendet wird.

5. Verfahren nach einem der vorhergehenden Ansprüche, dadurch gekennzeichnet, daß man $10^2$ bis $10^4$ U/l Glycerinkinase einsetzt.

6. Verfahren nach einem der vorhergehenden Ansprüche, dadurch gekennzeichnet, daß man wenigstens zwei Messungen in einem vorher bestimmten Zeitintervall vornimmt.

7. Verfahren nach einem der vorhergehenden Ansprüche, dadurch gekennzeichnet, daß man die Reaktion in gepufferter Lösung bei einem pH-Wert von 6,5 bis 8,5 durchgeführt.

8. Verfahren nach einem der vorhergehenden Ansprüche, dadurch gekennzeichnet, daß man gebildetes Glycerin-3-phosphat mit Glycerin-3-phosphatoxidase in Dihydroxyacetonphosphat und $H_2O_2$ überführt und letzteres bestimmt.

9. Verfahren nach Anspruch 8, dadurch gekennzeichnet, daß man zur Bestimmung des gebildeten Wasserstoff-Peroxids 4-Aminoantipyrin, Phenol, Peroxidase und GOOD-Puffer zusetzt.

10. Verfahren nach einem der Ansprüche 1 bis 7, dadurch gekennzeichnet, daß man gebildetes ADP bestimmt durch Umsetzung mit Phosphoenolpyruvat in Gegenwart von Pyruvatkinase unter Bildung von Pyruvat, welches mit NADH in Gegenwart von Lactatdehydrogenase zu Lactat und $NAD^+$ reagieren gelassen und die NADH-Abnahme bestimmt wird.

11. Verfahren nach einem der Ansprüche 1 bis 7, dadurch gekennzeichnet, daß gebildetes Glycerin-3-phosphat bestimmt wird durch Umsetzung mit $NAD^+$ in Gegenwart von Glycerinphosphatdehydrogenase unter Bildung von Dihydroxyacetonphosphat und NADH und letzteres mit Tetrazoliumsalz in Gegenwart von Diaphorase oxidiert wird unter Bildung eines Formazanfarbstoffs.

12. Verfahren nach einem der vorhergehenden Ansprüche, dadurch gekennzeichnet, daß man ein nichtionisches Detergenz und gegebenenfalls zusätzlich ein Detergenz der Cholsäure-Gruppe zusetzt.

13. Kombinationspräparat zur kinetischen Bestimmung von Glycerin, enthaltend Glycerinkinase, ATP und ein System zur Bestimmung von Glycerin-3-Phosphat oder ADP und gegebenenfalls eine Hydrolase, dadurch gekennzeichnet, daß es zusätzlich einen Zucker der allgemeinen Formel

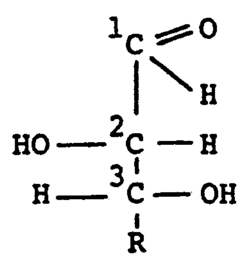

in der die C-Atome 2 und 3 D-threo-Konfiguration aufweisen und R einen Kohlenhydratrest mit 1 bis 3 Kohlenstoffatomen darstellt, bei dem eine OH-Gruppe auch durch ein Wasserstoffatom ersetzt sein kann, enthält.

14. Kombinationspräparat nach Anspruch 13, dadurch gekennzeichnet, daß es 1 bis 200 mMol/l Verbindung der allgemeinen Formel I enthält.

15. Kombinationspräparat nach Anspruch 13 oder 14, dadurch gkennzeichnet, daß es als Verbindung der allgemeinen Formel I L-Xylose enthält.

16. Kombinationspräparat nach einem der Ansprüche 13 bis 15, dadurch gekennzeichnet, daß es eine Glycerinkinase aus einem Mikroorganismus der Gattung Bacillus stearothermophilus enthält.

17. Kombinationspräparat nach einem der Ansprüche 13 bis 16, dadurch gekennzeichnet, daß es als System zur Bestimmung von Glycerin-3-phosphat Glycerinphosphatoxidase und ein System zur Bestimmung von $H_2O_2$ oder von Dihdyroxyacetonphosphat enthält.

18. Kombinationspräparat nach Anspruch 17, dadurch gekennzeichnet, daß das System zur Bestimmung von $H_2O_2$, 4-Aminoantipyrin, Phenol oder ein Derivat davon, Puffer und Detergenz enthält.

19. Kombinationspräparat nach einem der Ansprüche 13 bis 16, dadurch gekennzeichnet, daß das System zur Bestimmung von Glycerin-3-phosphat aus Glycerinphosphatdehydrogenase, $NAD^+$, Tetrazoliumsalz und Diaphorase besteht.

20. Kombinationspräparat nach einem der Ansprüche 13 bis 16, dadurch gekennzeichnet, daß das System zur Bestimmung von ADP aus Phosphoenolpyruvat, Pyruvatkinase, NADH und Lactatdehydrogenase besteht.

21. Kombinationspräparat nach einem der Ansprüche 13 bis 20, dadurch gekennzeichnet, daß es ein nichtionisches Detergenz allein oder zusammen mit einem Detergenz der Cholsäuregruppe enthält.

22. Kombinationspräparat nach den Ansprüche 17 bis 21, dadurch gekennzeichnet, daß es

$10^2$ bis $10^4$ U/l Glycerinkinase,

$10^2$ bis $10^4$ U/l Glycerinphosphatoxidase,

$10^3$ bis $2\times10^4$ U/l Cholesterin-Esterase,

$10^2$ bis $10^4$ U/l Peroxidase,

1 bis 20 mMol/l L-Xylose,
0,1 bis 1 mMol/l 4-Aminoantipyrin,
1 bis 10 mMol/l Phenol oder Phenol-Derivat,
1 bis 20 g/l nichtionisches Detergenz,
0 bis 15 mMol/l Detergenz der Cholsäuregruppe,
50 bis 200 mMol/l Puffer, pH 6,5 bis 8,5 enthält.

23. Kombinationspräparat nach einem der Ansprüche 13 bis 22, dadurch gekennzeichnet, daß es GOOD-Puffer enthält.

24. Kombinationspräparat nach einem der Ansprüche 13 bis 23, dadurch gekennzeichnet, daß es auf einem festen Träger imprägniert vorliegt.

**Revendications**

1. Procédé pour la détermination du glycérol sous forme libre ou liée, par réaction avec l'ATP en présence de glycérol kinase (GK) et éventuellement d'une hydrolase, avec formation de glycérol-3-phosphate et d'ADP et détermination de l'un de ces produits de réaction à l'aide d'au moins une réaction enzymatique subséquente, caractérisé en ce que la détermination est effectuée cinétiquement et en ce que pour ce faire la réaction avec l'ATP est rendue déterminante de la vitesse pour la réaction totale et qu'on la fait se dérouler selon le pseudopremier ordre, en ajoutant un sucre de formule générale I

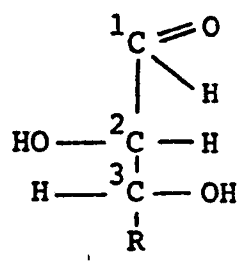

dans laquelle les atomes de C 2 et 3 présentent la configuration D-thréo et R représente un radical d'hydrate de carbone avec 1 à 3 atomes de carbone, dans lequel un groupe OH peut également être remplacé par un atome d'hydrogène.

2. Procédé selon la revendication 1, caractérisé en ce qu'on ajoute 1 à 200 mmol/l du composé de formule I.

3. Procédé selon la revendication 1 ou 2, caractérisé en ce que l'on utilise, comme composé de formule générale I, le L-xylose.

4. Procédé selon l'une des revendications précédentes, caractérisé en ce que l'on utilise une glycérol kinase provenant d'un microorganisme de l'espèce Bacillus stearothermophilus.

5. Procédé selon l'une des revendications précédentes, caractérisé en ce que l'on met en oeuvre $10^2$ à $10^4$ U/l de glycérol kinase.

6. Procédé selon l'une des revendications précédentes, caractérisé en ce qu'on effectue au moins deux mesures dans un intervalle de temps prédéterminé.

7. Procédé selon l'une des revendications précédentes, caractérisé en ce qu'on effectue la réaction en solution tamponnée à une valuer de pH de 6,5 à 8,5.

8. Procédé selon l'une des revendications précédentes, caractérisé en ce que l'on transforme le glycérol-3-phosphate formé, au moyen de glycérol-3-phosphate oxydase, en dihydroxyacétonephosphate et $H_2O_2$ et en ce qu'on détermine ce dernier.

9. Procédé selon la revendication 8, caractérisé en ce que, pour la détermination du peroxyde d'hydrogène formé, on ajoute de la 4-aminoantipyrine, du phénol, de la peroxydase et du tampon GOOD.

10. Procédé selon l'une des revendications 1 à 7, caractérisé en ce que, l'on détermine l'ADP formé par réaction avec du phosphoénol pyruvate en présence de pyruvate kinase avec formation de pyruvate, en ce qu'on fait réagir celui-ci avec du NADH en présence de lactate deshydrogénase pour donner du lactate et du $NAD^+$ et en ce qu'on détermine la diminution du NADH.

11. Procédé selon l'une des revendications 1 à 7, caractérisé en ce qu'on détermine le glycérol-3-phosphate formé par réaction avec du $NAD^+$ en présence de glycérol-phosphate deshydrogénase avec formation de dihydroxyacétonephosphate et de NADH et en ce qu'on oxyde ce dernier au moyen de sel de tétrazolium en présence de diaphorase avec formation d'un colorant formazan.

12. Procédé selon l'une des revendications précédentes, caractérisé en ce qu'on ajoute un détergent non ionique et éventuellement en plus un détergent du groupe de l'acide choliqie.

13. Préparation combinée pour la détermination cinétique du glycérol, contenant de la glycérol kinase,

de l'ATP et un système pour la détermination du glycérol-3-phosphate ou de l'ADP et éventuellement une hydrolase, caractérisée en ce qu'elle contient en outre un sucre de formule générale

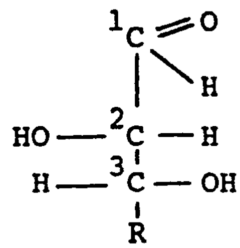

dans laquelle les atomes de C 2 et 3 présentent la configuration D-thréo et R représente un radical d'hydrate de carbone avec 1 à 3 atomes de carbone, dans lequel un groupe OH peut également être remplacé par un atome d'hydrogène.

14. Préparation combinée selon la revendication 13, caractérisée en ce qu'elle contient 1 à 200 mmol/l de composé de formule générale I.

15. Préparation combinée selon la revendication 13 ou 14, caractérisée en ce qu'elle contient, en tant que composé de formule générale I, du L-xylose.

16. Préparation combinée selon l'une des revendication 13 à 15, caractérisée en ce qu'elle contient une glycérol kinase provenant d'un microorganisme de l'espèce Bacillus stearothermophilus.

17. Préparation combinée selon l'une des revendication 13 à 16, caractérisée en ce qu'elle contient en tant que système pour la détermination du glycérol-3-phosphate de la glycérolphosphate oxydase, et un système pour la détermination de $H_2O_2$ ou du dihydroxyacétonephosphate.

18. Préparation combinée selon la revendication 17, caractérisée en ce que le système pour la détermination de $H_2O_2$ contient de la 4-aminoantipyrine, du phénol, ou un dérivé de celui-ci, du tampon et du détergent.

19. Préparation combinée selon l'une des revendications 13 à 16, caractérisée en ce que le système pour la détermination du glycérol-3-phosphate est constitué de glycérolphosphate deshydrogénase, de $NAD^+$, de sel de tétrazolium et de diaphorase.

20. Préparation combinée selon l'une des revendications 13 à 16, caractérisée en ce que le système pour la détermination de l'ADP est constitué de phosphoénolpyruvate, de pyruvate kinase, de NADH et de lactate deshydrogénase.

21. Préparation combinée selon l'une des revendications 13 à 20, caractérisée en ce qu'elle contient un détergent non ionique seul ou ensemble avec un détergent du groupe de l'acide cholique.

22. Préparation combinée selon les revendications 17 à 21, caractérisée en ce qu'elle contient
$10^2$ à $10^4$ U/l de glycérol kinase,
$10^2$ à $10^4$ U/l de glycérolphosphate oxydase,
$10^3$ à $2\times10^4$ U/l de cholestérol-estérase,
$10^2$ à $10^4$ U/l de peroxydase,
1 à 20 mmol/l de L-xylose,
0,1 à 1 mmol/l de 4-aminoantipyrine,
1 à 10 mmol/l de phénol ou de dérivé du phénol,
1 à 20 g/l de détergent non ionique,
0 à 15 mmol/l de détergent du groupe de l'acide cholique,
50 à 200 mmol/l de tampon, pH 6,5 à 8,5.

23. Préparation combinée selon l'une des revendications 13 à 22, caractérisée en ce qu'elle contient du tampon GOOD.

24. Préparation combinée selon l'une des revendications 13 à 23, caractérisée en ce qu'elle se présente imprégnée sur un support solide.

**Claims**

1. Process for the determination of glycerol in free or bound form by reaction with ATP in the presence of glycerol kinase (GK) and possibly a hydrolase with formation of glycerol-3-phosphate and ADP and determination of one of these reaction products with the help of at least one subsequent enzymatic reaction, characterised in that the determination is carried out kinetically and, for this purpose, the reaction

with ATP is made rate-determining for the whole reaction and is allowed to proceed according to the pseudo-first order, in that one adds a sugar of the general formula I

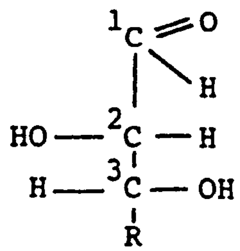

in which the C-atoms 2 and 2 have the D-threo configuration and R represents a carbohydrate radical with 1 to 3 carbon atoms, in which one OH group can also be replaced by a hydrogen atom.

2. Process according to claim 1, characterised in that one adds 1 to 200 mMole/l of the compound of formula I.

3. Process according to claim 1 or 2, characterised in that one uses L-xylose as compound of the general formula I.

4. Process according to one of the preceding claims, characterised in that one uses a glycerol kinase from a micro-organism of the species Bacillus stearothermophilus.

5. Process according to one of the preceding claims, characterised in that one uses $10^2$ to $10^4$ U/l of glycerol kinase.

6. Process according to one of the preceding claims, characterised in that one carries out at least two measurements in a predetermined time interval.

7. Process according to one of the preceding claims, characterised in that one carries out the reaction in buffered solution at a pH value of 6.5 to 8.5.

8. Process according to one of the preceding claims, characterised in that one converts glycerol-3-phosphate formed with glycerol-3-phosphate oxidase into dihdyroxyacetone phosphate and $H_2O_2$ and determines the latter.

9. Process according to claim 8, characterised in that one adds 4-aminoantipyrine, phenol, peroxidase and GOOD buffer for the determination of the hydrogen peroxide formed.

10. Process according to one of claims 1 to 7, characterised in that one determines ADP formed by reaction with phosphenol pyruvate in the presence of pyruvate kinase with the formation of pyruvate which is allowed to react with NADH in the presence of lactate dehydrogenase to give lactate and $NAD^+$ and the NADH decrease is determined.

11. Process according to one of claims 1 to 7, characterised in that glycerol-3-phosphate formed is determined by reaction with $NAD^+$ in the presence of glycerol phosphate dehydrogenase with the formation of dihydroxyacetone phosphate and NADH and the latter is oxidised with tetrazolium salt in the presence of diaphorase with the formation of a formazane coloured material.

12. Process according to one of the preceding claims, characterised in that one adds a non-ionic detergent and possibly additionally a detergent of the cholic acid group.

13. Combination preparation for the kinetic determination of glycerol, containing glycerol kinase, ATP and a system for the determination of glycerol-3-phosphate or ADP and possibly a hydrolase, characterised in that it additionally contains a sugar of the general formula

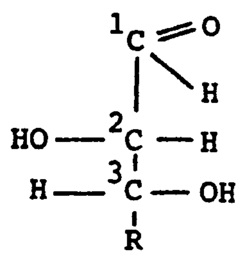

in which the C-atoms 2 and 3 have the D-threo configuration and R represents a carbohydrate radical with 1 to 3 carbon atoms, in which one OH group can also be replaced by a hydrogen atom.

14. Combination preparation according to claim 13, characterised in that it contains 1 to 200 mMole/l of compound of the general formula I.

15. Combination preparation according to claim 13 or 14, characterised in that it contains L-xylose as compound of the general formula I.

16. Combination preparation according to one of claims 13 to 15, characterised in that it contains a glycerol kinase from a micro-organism of the species Bacillus stearothermophilus.

17. Combination preparation according to one of claims 13 to 16, characterised in that, as system for the determination of glycerol-3-phosphate, it contains glycerol phosphate oxidase and a system for the

determination of $H_2O_2$ or of dihydroxyacetone phosphate.

18. Combination preparation according to claim 17, characterised in that the system for the determination of $H_2O_2$ contains 4-aminoantipyrine, phenol or a derivative thereof, buffer and detergent.

19. Combination preparation according to one of claims 13 to 16, characterised in that the system for the determination of glycerol-3-phosphate consists of glycerol phosphate dehydrogenase, $NAD^+$, tetrazolium salt and diaphorase.

20. Combination preparation according to one of claims 13 to 16, characterised in that the system for the determination of ADP consists of phosphoenol pyruvate, pyruvate kinase, NADH and lactate dehydrogenase.

21. Combination preparation according to one of claims 13 to 20, characterised in that it contains a non-ionic detergent alone or together with a detergent of the cholic acid group.

22. Combination preparation according to one of claims 17 to 21, characterised in that it contains:

$10^2$ to $10^4$ U/l glycerol kinase,
$10^2$ to $10^4$ U/l glycerol phosphate oxidase,
$10^3$ to $2\times10^4$ U/l cholesterol esterase,
$10^2$ to $10^4$ U/l peroxidase,
1 to 20 mMole/l L-xylose,
0.1 to 1 mMole/l 4-aminoantipyrine,
1 to 10 mMole/l phenol or phenol derivative,
1 to 20 g/l non-ionic detergent,
0 to 15 mMole/l detergent of the cholic acid group,
50 to 200 mMole/l buffer, pH 6.5 to 8.5.

23. Combination preparation according to one of claims 13 to 22, characterised in that it contains GOOD buffer.

24. Combination preparation according to one of claims 13 to 23, characterised in that it is present impregnated on to a solid carrier.

FIG. 1

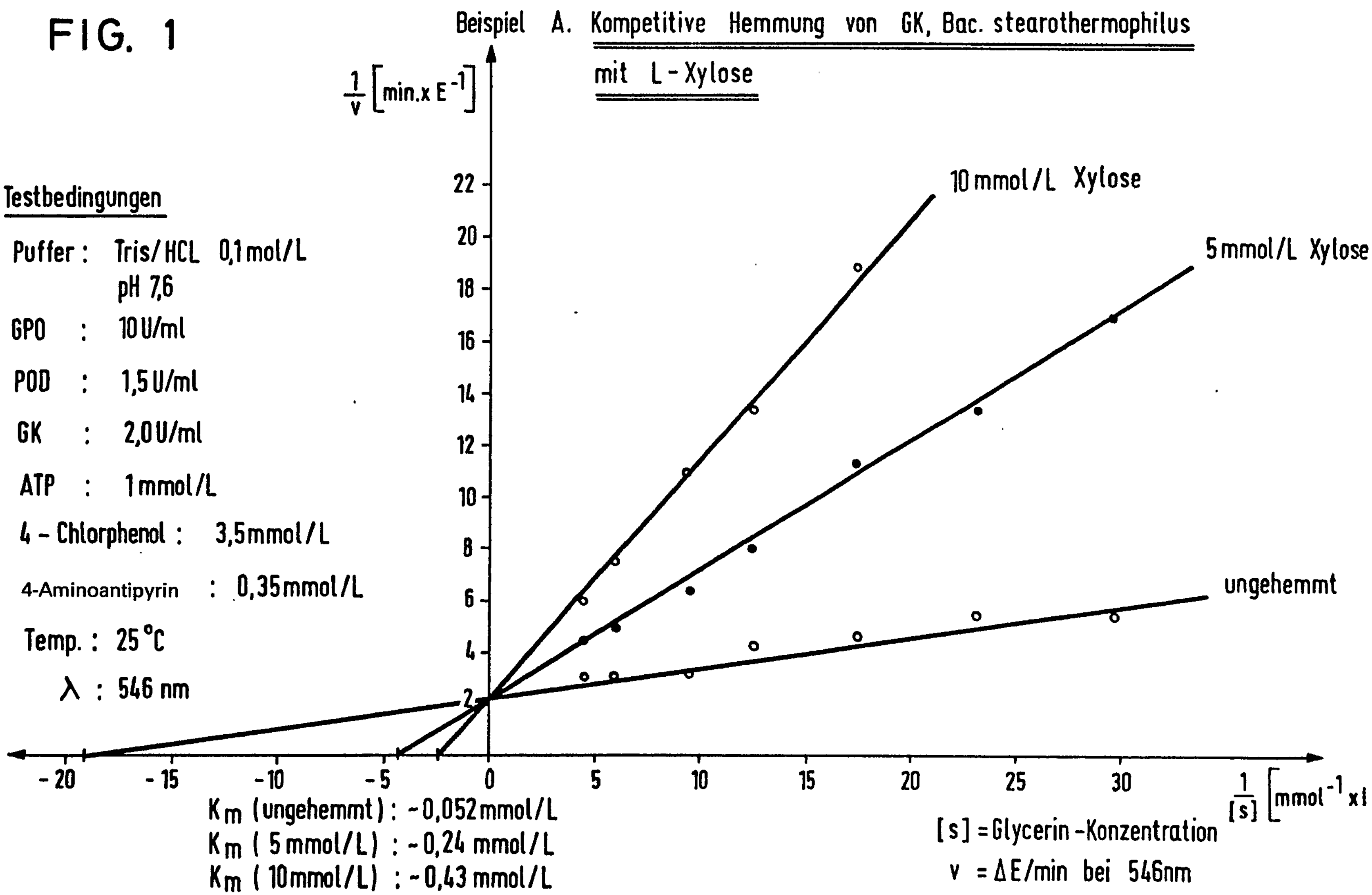
Beispiel A. Kompetitive Hemmung von GK, Bac. stearothermophilus
mit L-Xylose
1/v [min. x E^-1]
Testbedingungen
Puffer : Tris/HCL 0,1 mol/L
pH 7,6
GPO : 10 U/ml
POD : 1,5 U/ml
GK : 2,0 U/ml
ATP : 1 mmol/L
4 - Chlorphenol : 3,5 mmol/L
4-Aminoantipyrin : 0,35 mmol/L
Temp. : 25 °C
λ : 546 nm
10 mmol/L Xylose
5 mmol/L Xylose
ungehemmt
22
20
18
16
14
12
10
8
6
4
2
-20
-15
-10
-5
0
5
10
15
20
25
30
1/[s] [mmol^-1 x l]
Km (ungehemmt) : -0,052 mmol/L
Km ( 5 mmol/L) : -0,24 mmol/L
Km ( 10 mmol/L) : -0,43 mmol/L
[s] = Glycerin-Konzentration
v = ΔE/min bei 546 nm

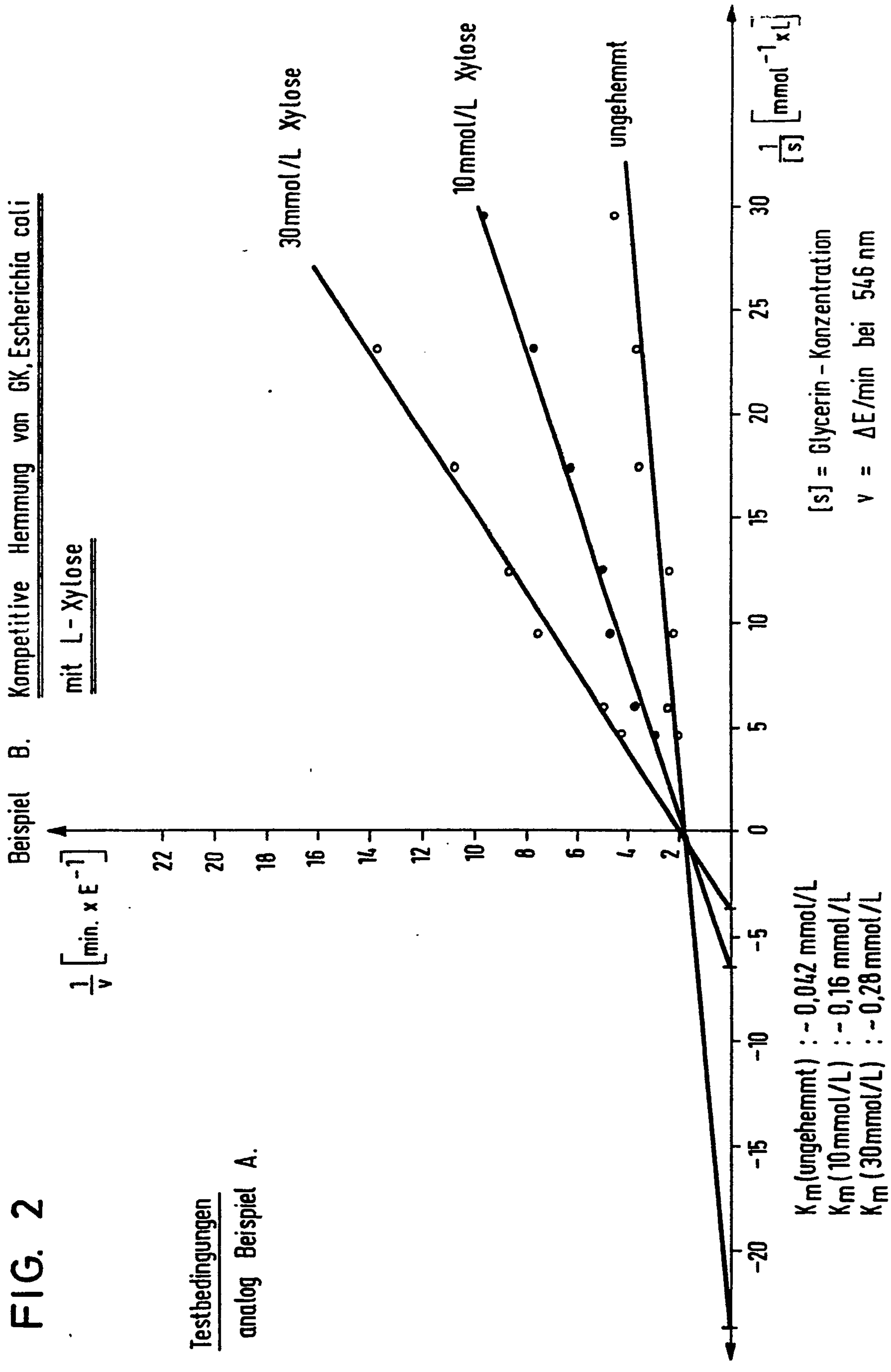
Beispiel B. Kompetitive Hemmung von GK, Escherichia coli
mit L-Xylose
Testbedingungen
analog Beispiel A.
1/v [min. x E⁻¹]
22
20
18
16
14
12
10
8
6
4
2
30mmol/L Xylose
10mmol/L Xylose
ungehemmt
-20
-15
-10
-5
0
5
10
15
20
25
30
1/[s] [mmol⁻¹ x L]
[s] = Glycerin-Konzentration
v = ΔE/min bei 546 nm
Km(ungehemmt) : - 0,042 mmol/L
Km(10mmol/L) : - 0,16 mmol/L
Km(30mmol/L) : - 0,28 mmol/L

FIG. 2